# EUROPEAN PATENT APPLICATION

(11) **EP 4 830 642 A1**
(43) Date of publication of application: **28.10.2026**
(21) Application number: 24908139.9
(22) Date of filing: 20.12.2024
(51) Int. Cl.: A61B 17/00

(54) **POWDER APPLICATOR**

(30) Priority: 21.12.2023 KR 20230188704
(71) Applicant: SAMYANG BIOPHARM CORPORATION, Seongnam-si Gyeonggi-do 13488 (KR)
(72) Inventor: KIM, Hyung Hwan, Seongnam-si Gyeonggi-do 13504 (KR); YOON, Hye Sung, Seongnam-si Gyeonggi-do 13544 (KR); LEE, Joo Hang, Seongnam-si Gyeonggi-do 13263 (KR)
(74) Representative: Gleiss Große Schrell und Partner mbB
(86) International application number: PCT/KR2024/020790
(87) International publication number: WO 2025/135857

(57) **Abstract**

The present invention relates to a powder applicator comprising: a first body part including mixing chambers communicating with each other, an air reservoir filled with air, and an air supply passage disposed between the mixing chambers and the air reservoir; a second body part disposed on a side surface of the first body part and supplying powder to the mixing chambers; a pneumatic pressure generation part positioned at the other end portion of the first body part to generate air pressure in the air filled in the air reservoir and supply the air to the mixing chambers; a filter disposed inside the air reservoir and completely covering the air supply passage to block the movement of the powder between the mixing chambers and the air reservoir while allowing the movement of the air; and a discharge port disposed at one end portion of the mixing chamber to discharge the powder, wherein the powder disposed in the mixing chamber is transported by the flow of air by the air pressure generated by the pneumatic pressure generation part and discharged through the discharge port.

## Description

### [TECHNICAL FIELD]

The present invention relates to a powder applicator.

### [BACKGROUND ART]

During surgical operations, various bleeding situations occur. For severe bleeding, primary hemostatic measures such as sutures, clips, or staplers are employed. For additional small amounts of bleeding, sheets, gauze, powder, and gel-type hemostatic agents are used for the purpose of auxiliary hemostasis.

Unlike other types of hemostatic agents, powder-type hemostatic agents are used in an applicator, so they can be used for bleeding in deep and narrow areas that are difficult to reach with hands. In addition, since there are no shape restrictions, they have the advantage of being able to be applied in various areas regardless of the bleeding site and area. However, since there are no shape restrictions, in order to accurately apply the powder hemostatic agent to the affected area, it must be used in a form filled in a powder spray applicator.

For such powder spray applicators, the amount used must be adjusted according to the amount and area of bleeding, so the applicator should allow multiple spray operations, and each spray operation should deliver a consistent amount of powder so that the user can predict the amount of powder being applied.

### [CONTENTS OF THE INVENTION]

### [PROBLEMS TO BE SOLVED]

The purpose of the present invention is to provide a powder applicator that is capable of multiple spray operations with providing improved uniformity in the amount of powder dispensed and accuracy of spraying timing.

The technical problems of the present invention are not limited to the technical problems mentioned above, and other technical problems that are not mentioned will be clearly understood by those skilled in the art to which the present invention belongs from the description below.

### [TECHNICAL MEANS]

A powder applicator according to one embodiment of the present invention comprises: a first body portion comprising an interconnected mixing chamber, an air reservoir configured to contain air, and an air supply passage disposed between the mixing chamber and the air reservoir; a second body portion disposed on a side surface of the first body portion and supplying powder to the mixing chamber; an air pressure generating unit disposed at the other end of the first body portion, generating air pressure in the air filled in the air reservoir and supplying air to the mixing chamber; a filter disposed inside the air reservoir, completely covering the air supply passage, and configured to prevent themovement of powder between the mixing chamber and the air reservoir and to allow movement of air; and a discharge port disposed at one end of the mixing chamber through which the powder is discharged, wherein the powder contained in the mixing chamber is transported by an airflow generated by the air pressure of the air pressure generating unit and discharged through the discharge port.

The first body portion may comprise a first main body comprising the mixing chamber, the air reservoir, and the air supply passage; a coupling portion disposed at one end of the first main body and having a structure for coupling with the discharge port; and a handle portion disposed adjacent to the coupling portion and having a shape protruding from a side surface of the first main body.

The second body portion may comprise a second main body comprising a powder reservoir; a closing member closing the other end of the second main body; a plunger disposed inside the second main body and configured to seal between the powder reservoir and a space adjacent to the other end of the second main body; and a first spring having one end coupled to the plunger and the other end coupled to the closing member.

The second main body may further comprise a powder supply chamber in communication with the powder reservoir, wherein the powder supply chamber is disposed inside the mixing chamber, the powder contained in the powder reservoir is supplied to the mixing chamber through the powder supply chamber, and the planar area of the opening through which the powder is supplied from the powder supply chamber to the mixing chamber may be smaller than the planar area of the powder reservoir.

The plunger may be biased by the first spring to apply pressure to the powder contained in the powder reservoir.

The air pressure generating unit may comprise a pump having a structure that folds in a corrugated manner when an external force is applied; and a first support member fixedly coupled together with the pump to the other end of the first body portion.

The air pressure generating unit may further comprise a second spring disposed between the first support member and the other end of the pump; and a second support member disposed between the second spring and the pump to prevent the second spring from bending when an external force is applied to the pump.

The air pressure generating unit may be detachable from the first body portion.

The powder applicator may further comprise a barrier member coupled to the discharge port to block contact between the powder and the external environment.

The discharge port may comprise a powder trap disposed adjacent to the mixing chamber to prevent unintended discharge of the powder.

The powder trap may comprise an arrow-shaped passage facing in a direction opposite to a powder discharge direction, wherein a portion of the passage adjacent to the powder reservoir may face in the direction opposite to the direction in which the powder is discharged. The first body portion and the second body portion may be manufactured separately and may be combinable.

Specific details of other embodiments are included in the detailed description and drawings.

### [EFFECT OF THE INVENTION]

In the case of the powder applicator according to the embodiments of the present invention, the powder contained therein may be sprayed multiple times, and at the same time, the uniformity in the amount of powder sprayed and the accuracy of the spray timing are improved.

The effects according to the embodiments of the present invention are not limited to the contents exemplified above, and more diverse effects are included in this specification.

### [Brief Description of the Figures]

Fig. 1 is a perspective view of a powder applicator according to one embodiment of the present invention.
Fig. 2 is a side view of a powder applicator according to one embodiment of the present invention.
Fig. 3 is a plan view of a powder applicator according to one embodiment of the present invention.
Fig. 4 is an exploded view of a powder applicator according to one embodiment of the present invention.
Figs. 5 to 7 are cross-sectional views of a powder applicator according to one embodiment of the present invention, illustrating an operating method of the powder applicator.

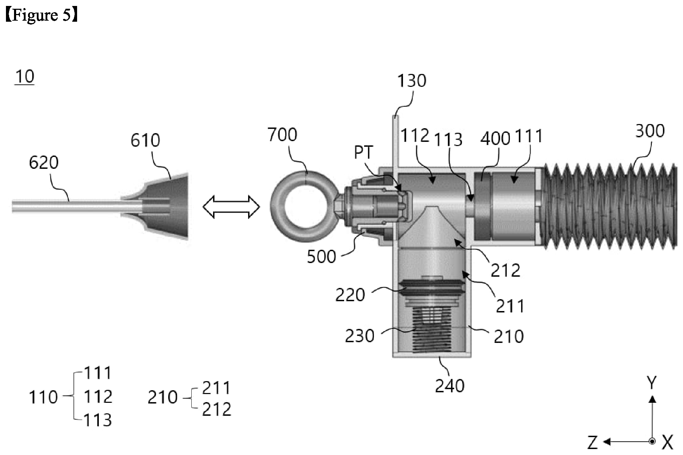

Fig. 5 illustrates a state in which a barrier member is coupled to a discharge port before use of a powder applicator according to one embodiment of the present invention is used.

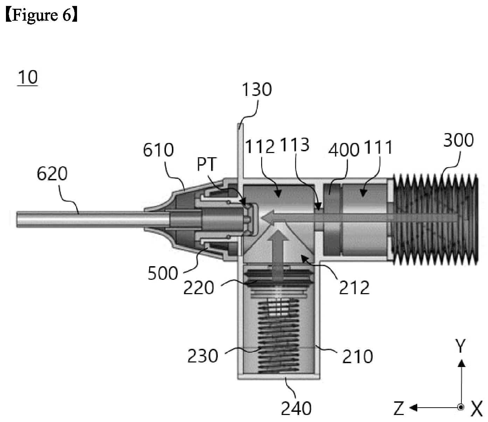

Fig. 6 illustrates a state in which an external force is applied to a powder applicator according to one embodiment of the present invention.

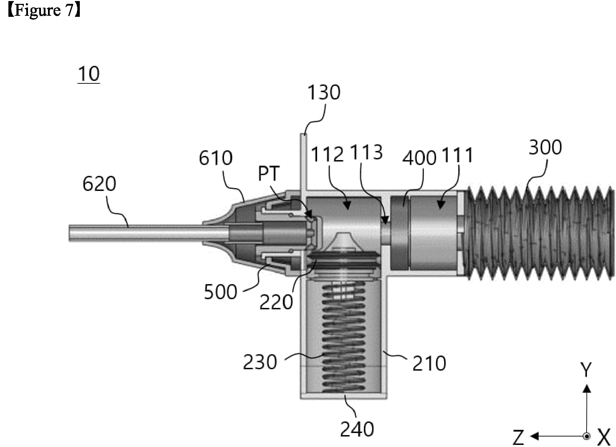

Fig. 7 illustrates a state after all powder contained in a powder applicator according to one embodiment of the present invention has been discharged.

### [CONCRETE MODE FOR CARRYING OUT THE INVENTION]

The advantages and features of the present invention, and methods for achieving them, will become apparent upon reference to the embodiments described in detail with reference to the accompanying drawings. However, the invention is not limited to the embodiments disclosed herein, but will be embodied in many different forms, and these embodiments are provided only to make the disclosure of the present invention complete and to fully inform a person having ordinary skill in the art to which the present invention belongs of the scope of the invention, and the present invention is defined only by the scope of the claims.

Throughout the specification, like reference numerals refer to like components. The shapes, sizes, proportions, angles, numbers, etc. disclosed in the drawings to illustrate embodiments are exemplary and do not limit the invention to those shown.

Although the terms first, second, and the like are used to describe various components, the components are not limited by these terms. Thus, a first component referred to herein may also be a second component within the technical idea of the present invention.

The individual features of the various embodiments of the present invention may be partially or wholly combined or combined with each other, and various technical connections are possible, and the embodiments may be implemented independently of each other or implemented together in a related relationship.

Specific embodiments will be described below with reference to the accompanying drawings.

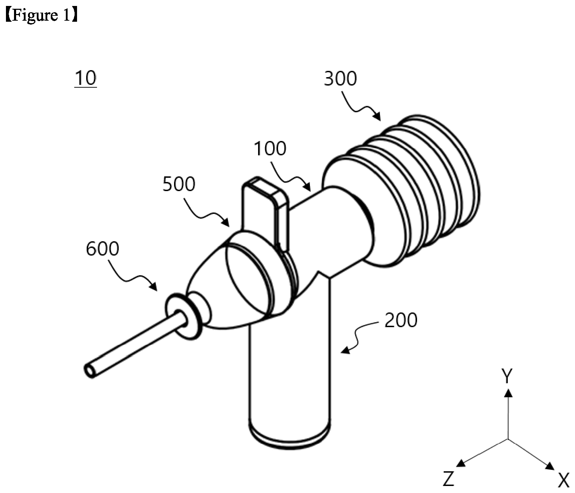

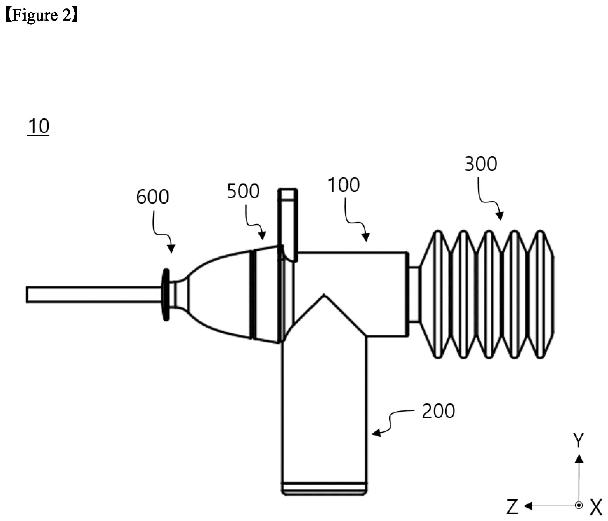

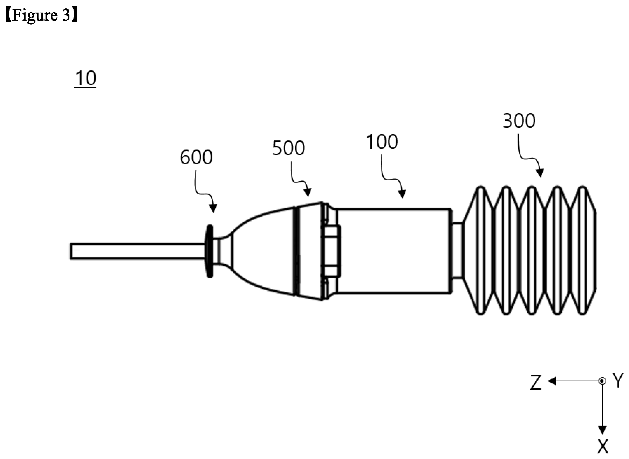

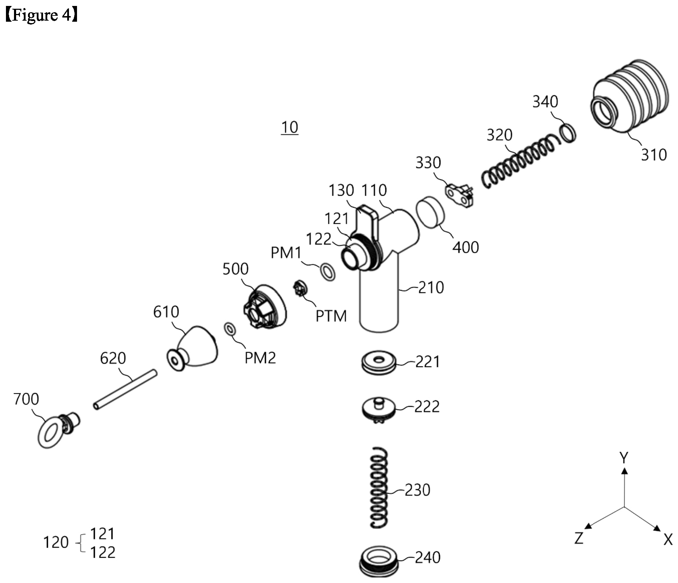

Fig. 1 is a perspective view of a powder applicator according to one embodiment of the present invention. Fig. 2 is a side view of a powder applicator according to one embodiment of the present invention. Fig. 3 is a plan view of a powder applicator according to one embodiment of the present invention. Fig. 4 is an exploded view of a powder applicator according to one embodiment of the present invention.

First, in order to more clearly explain the technical idea of the present invention, a coordinate system defined in a first direction (X), a second direction (Y) perpendicular to the first direction (X) and in the same plane, and a third direction (Z) perpendicular to the plane of the first direction (X) and the second direction (Y) is described. The first direction (X), the second direction (Y), and the third direction (Z) are intended to explain directions related to the structure and operation method of the powder applicator (10), and should be understood to mean relative directions, and the embodiments are not limited to the mentioned directions.

For example, when viewed from the front, the first direction (X) may correspond to the horizontal direction of the powder applicator (10), the second direction (Y) may correspond to the vertical direction of the powder applicator (10), and the third direction (Z) may correspond to the height direction of the powder applicator (10).

Referring to Figs. 1 to 4, a powder applicator (10) according to one embodiment may comprise a first body portion (100), a second body portion (200), an air pressure generating unit (300), a filter (400), and a discharge port (500), and may optionally further comprise an spray member (600) and/or a barrier member (700). In addition, the powder applicator (10) may further comprise a plurality of sealing members (PM1, PM2) to provide airtightness between each component.

For example, the powder applicator (10) may comprise a first body portion (100) and a second body portion (200) that are coupled to each other and form the main outer shape, an air pressure generating unit (300) disposed at the other end of the first body portion (100) in the third direction (Z) for generating air pressure in the air filled in the first body portion (100), a filter (400) disposed inside the first body portion (100) that is configured to allow the flow of air but to prevent the flow of powder, a discharge port (500) disposed at one end of the first body portion (100) in the third direction (Z) through which the powder disposed in the first body portion (100) is discharged by the air pressure, an spray member (600) mounted on the discharge port (500) for spraying the powder discharged from the discharge port (500), and a barrier member (700) mounted on the discharge port (500) instead of the spray member (600) to block powder from being discharged.

The first body portion (100) may comprise a first main body (110), a coupling portion (120), and a handle portion (130). The first main body (110), the coupling portion (120), and the handle portion (130) may be integrally formed. The first main body (110) may have a cylindrical shape with a side surface extending in the third direction (Z). However, the present invention is not limited thereto, and the first main body (110) may have an elliptical shape or a polygonal shape such as a triangle, a square, or a pentagon on the X-Y plane.

In the third direction (Z), a discharge port (500) is disposed at one end of the first main body (110), and an air pressure generating unit (300) may be disposed at the other end. For example, a coupling portion (120) may be disposed at one end of the first main body (110) to be coupled to the discharge port (500), and an opening and a structure for coupling with the air pressure generating unit (300) may be formed at the other end so that the air pressure generating unit (300) is coupled thereto.

In addition, an opening and a coupling structure configured to couple with the second main body (210) may be formed on the side surface of the first main body (110). The size and shape of the opening and the coupling structure formed on the side surface of the first main body (110) may be variously determined according to the size and shape of the second main body (210).

As illustrated in Figs. 5 to 7, the first main body (110) may comprise an air reservoir (111), a mixing chamber (112), and an air supply passage (113) disposed between the air reservoir (111) and the mixing chamber (112). The air reservoir (111), the air supply passage (113), and the mixing chamber (112) may be sequentially arranged in the third direction (Z). The air reservoir (111) and the mixing chamber (112) may communicate with each other through the air supply passage (113).

In the third direction (Z), the air supply passage (113) is positioned on one side of the air reservoir (111), and the air pressure generating unit (300) may be disposed on the other side. The air reservoir (111) may be defined as an empty space configured to contain air. A filter (400) may be disposed inside the air reservoir (111) adjacent to the air supply passage

(113). In the third direction (Z), the discharge port (500) is disposed on one side of the mixing chamber (112), and the air supply passage (113) is positioned on the other side. In addition, as will be described below, the mixing chamber (112) may communicate with the powder reservoir (211) of the second main body (210), or with the powder reservoir (211) and the powder supply chamber (212). The mixing chamber (112) may be a space in which the powder contained in the powder reservoir (211) and the air contained in the air reservoir (111) are each supplied and mixed.

The coupling portion (120) has a structure configured to couple with the discharge port (500) on its outer peripheral surface, and may be formed adjacent to one end of the first main body (110) in the third direction (Z). The structure of the coupling portion (120) may be variously designed according to the structure of the discharge port (500).

For example, the coupling portion (120) may comprise a first sub-coupling portion (121) and a second sub-coupling portion (122). The first sub-coupling portion (121) has a shape protruding from the first main body (110) in the third direction (Z), and may have a structure for fixed coupling with the discharge port (500). As a non-limiting example, a screw thread may be formed on the outer peripheral surface of the first sub-coupling portion (121).

The second sub-coupling portion (122) has a shape protruding from the first sub-coupling portion (121) in the third direction (Z), and may have a cylindrical shape with a planar area on the X-Y plane smaller than that of the first sub-coupling portion (121). The internal structure of the discharge port (500), as will be described below, may be fitted into and coupled to the inner peripheral surface of the second sub-coupling portion (122).

The handle portion (130) may have a structure that supports the powder applicator (10) when the user uses the powder applicator (10) and an external force is applied in the third direction (Z). The handle portion (130) may have a structure that protrudes from the side surface of the first main body (110).

For example, the handle portion (130) may have a shape protruding in the second direction (Y) from the side surface of the first main body (110) adjacent to the coupling portion (120). In addition, the handle portion (130) may have a structure extending in the direction opposite to the second main body (210) along the second direction (Y). However, the shape of the handle portion (130) is not limited thereto, and may have various shapes for a user to support the powder applicator (10) in the third direction (Z).

The second body portion (200) may comprise a second main body (210), a plunger (220), a first spring (230), and a closing member (240). The plunger (220) and the first spring (230) are disposed in the internal space of the second main body (210), and the closing member (240) may close the other end of the second main body (210) in the second direction (Y).

The second main body (210) may have a cylindrical outer shape whose side surface extends along the second direction (Y), but is not limited thereto. As another example, the second main body (210) may have an elliptical shape or a polygonal shape such as a triangle, a square, or a pentagon on the X-Z plane. The second main body (210) may be coupled to the side surface of the first main body (110).

In the second direction (Y), one end of the second main body (210) has a structure for fixed coupling with the first main body (110), and the other end may have a structure configured to couple with the closing member (240). In the present specification, a region of the second main body (210) inserted into the side surface of the first main body (110) (i.e., the outer shape of the powder supply chamber (212)) is exemplified as having a conical shape, but is not limited thereto.

The second main body (210) according to one embodiment may comprise a powder reservoir (211). The powder reservoir (211) is an internal space of the second main body (210), and may be a space defined by one end of the second main body (210) and the plunger (220) along the second direction (Y). The powder reservoir (211) may contain powder, and the powder contained in the powder reservoir (211) may be supplied to the mixing chamber (112) by the elastic force of the first spring (230) applied in the second direction (Y) through the plunger (220).

In addition, a flow-prevention structure (not shown) for preventing the plunger (220) from entering the mixing chamber (112) may be formed at one end of the second main body (210) in the second direction (Y). The size, shape, and arrangement structure of the flow-prevention structure may be variously designed as understood by those skilled in the art.

In another embodiment, the second main body (210) may further comprise a powder supply chamber (212) in communication with the powder reservoir (211). In this case, the powder reservoir (211) and the powder supply chamber (212) may be sequentially arranged in the second direction (Y).

The powder reservoir (211) and the powder supply chamber (212) may be a single space located inside the second main body (210), and may be distinguished based on the first main body (110). For example, the powder supply chamber (212) may be defined as a space, among the internal space of the second main body (210), where the second main body (210) is inserted into the mixing chamber (112) of the first main body (110), and the powder reservoir (211) may be defined as the remaining space.

The powder supply chamber (212) according to the present embodiment may be implemented in a conical shape comprising an opening at its end in the second direction (Y), as illustrated in Figs. 5 to 7, to supply powder to the mixing chamber (112). However, the present invention is not limited thereto, and the shape of the powder supply chamber (212) may have various shapes as understood by those skilled in the art as described below.

The powder applicator (10) according to the present embodiment comprises a powder supply chamber (212) disposed between the mixing chamber (112) and the powder reservoir (211), so that compared to the case where the powder reservoir (211) directly communicates with the mixing chamber (112), the powder supply position is located inside the mixing chamber (112) and a planar area of the opening through which the powder is supplied may be smaller than a planar area of the powder reservoir (211). Accordingly, by reducing the resistance between the air supplied to the mixing chamber (112) to discharge the powder and the powder supplied to the mixing chamber (112), it can be advantageous in improving the spray performance of the powder applicator (10).

The plunger (220) is disposed inside the second main body (210), and can move toward the second direction (Y) inside the second main body (210) by the elastic force of the first spring (230) in the second direction (Y). The plunger (220) can apply pressure to the powder contained in the powder reservoir (211) and the powder supply chamber (212) in the second direction (Y). The plunger (220) may comprise a packing member (221) and a connecting member (222).

The packing member (221) can seal between the powder reservoir (211) and a space adjacent to the other end of the second main body (210) in the second direction (Y) (i.e., a space where the plunger (220) and the first spring (230) are disposed). The packing member (221) may have a diameter corresponding to the inner diameter of the second main body (210). The size and shape of the packing member (221) may be variously set to seal the internal space of the second main body (210). For example, when the second main body (210) has a polygonal shape on the X-Z plane, the packing member (221) may also have a corresponding polygonal shape.

The packing member (221) can have a sealing performance to the extent that foreign substances such as air or dust from the direction opposite to the second direction (Y) do not flow into the powder contained in the powder reservoir (211) and the powder does not leak.

In addition, the packing member (221) can apply pressure to the powder contained in the powder reservoir (211) by the elastic force transmitted from the first spring (230), but can have a rigidity that does not deform its shape. For example, the packing member (221) may be made of silicon.

The connecting member (222) may have a structure for connecting the packing member (221) to the first spring (230). For example, if the packing member (221) is implemented as a circular ring having a circular shape when viewed in the second direction (Y) and including an opening in the center, the connecting member (222) may have a structure in which one end in the second direction (Y) is fitted into the opening of the packing member (221), and the other end is coupled with the first spring (230). The connecting member (222) may have substantially the same size and shape as the packing member (221) in the second direction (Y).

The connecting member (222) may have a function of sealing the powder contained in the powder reservoir (211) similar to the packing member (221). The connecting member (222) may be composed of substantially the same material as the packing member (221), but is not limited thereto.

In the present specification, the packing member (221) and the connecting member (222) are exemplified as being formed as separate components and combined, but the present invention is not limited thereto, and the packing member (221) and the connecting member (222) may be formed as one piece.

The first spring (230) is arranged to be elastic in the second direction (Y), and one end in the second direction (Y) is fixed to the plunger (220), and the other end can be fixed to the closing member (240). The elastic coefficient of the first spring (230) can be set so that the plunger (220) applies pressure to the powder contained in the powder reservoir (211) to prevent formation of an empty space from being formed inside the powder reservoir (211), as understood by those skilled in the art according to the teachings of the present invention.

Furthermore, the elastic coefficient of the first spring (230) may be set according to the amount of powder discharged by the powder applicator (10). In addition, the diameter, length, and thickness of the first spring (230) may be determined according to the elastic coefficient of the first spring (230) and design details of other components (e.g., the width of the second main body (210) in the second direction (Y), the area on the X-Z plane, etc.).

The closing member (240) may close the other end of the second main body (210) in the second direction (Y). In addition, the closing member (240) may fix the other end of the first spring (230). For example, when a screw thread is formed on the inner peripheral surface of the other end of the second main body (210), the closing member (240) may comprise a corresponding screw thread for coupling and fixing to the other end of the second main body (210), and may comprise a structure in which the other end of the first spring (230) is engaged and fitted for fixing. However, the shape and size of the closing member (240) may be variously designed according to the shape and size of the second main body (210) and the first spring (230).

In this way, the powder applicator (10) according to one embodiment is implemented such that the second body portion (200) contains powder and the air reservoir (111) and air pressure generating unit (300) for supplying air for transporting the powder are separately implemented, so that the resistance can be reduced compared to a case where air must pass through all the powder corresponding to the total discharge amount of the powder applicator (10), which can be advantageous in improving the powder spray performance.

In addition, the powder applicator (10) comprises the first spring (230) that applies pressure to the powder reservoir (211) of the second main body (210) contains powder, which can be advantageous in providing uniform spray performance even when spraying a plurality of times.

Meanwhile, in the present specification, it is exemplified that the second body portion (200) is implemented integrally with the first body portion (100), but the present invention is not limited thereto. A powder applicator according to another embodiment may be implemented such that the second body portion (200) is manufactured separately from the first body portion (100) and combined with each other for use. In this case, by variously modifying the size of the second body portion (200) to be coupled to the first body portion (100) (e.g., the width of the second main body (210) in the second direction (Y)) and the elastic coefficient of the first spring (230) included in the second body portion (200), it is possible to control the total discharge amount of powder without having to change the overall size of the powder applicator.

The air pressure generating unit (300) may be disposed at the other end of the first body portion (100) in the third direction (Z). The air pressure generating unit (300) may receive an external force applied in the third direction (Z) to generate air pressure. Hereinafter, for the purpose of simplifying the cost, manufacturing, and driving of the powder applicator (10), the air pressure generating unit (300) according to one embodiment will be mainly described as comprising a bellows-shaped pump (310) and being manually operated by a user, but the present invention is not limited thereto. In another embodiment, the air pressure generating unit (300) may be implemented not only with various types of manual pumps, but also with an automatic pump electrically driven by a battery.

The air pressure generating unit (300) may comprise a pump (310), a second spring (320), a first support member (330), and a second support member (340). The pump (310) may be implemented as a bellows pump having a structure that folds in a corrugated manner when an external force is applied in the third direction (Z). One end of the pump (310) in the third direction (Z) may be fixedly coupled together with the first support member (330) to the other end of the first main body (110). One end of the pump (310) may have a structure for fixed coupling to the other end of the first main body (110), and the structure thereof may be implemented according to the shape of the other end of the first main body (110).

The first support member (330) is fixed to the other end of the first main body (110) in the third direction (Z), and may fix one end of the second spring (320). For example, as illustrated in Figs. 5 to 7, the first support member (330) is fixedly coupled to the opening and coupling structure formed at the other end of the first main body (110), and has a structure protruding in the direction opposite to the third direction (Z), thereby fixing one end of the second spring (320).

The second spring (320) and the second support member (340) may be disposed inside the pump (310). The second spring (320) is arranged to be elastic in the third direction (Z), and one end in the third direction (Z) is fixed to the first support member (330), and the other end may be fixed to the second support member (340). The second spring (320) is compressed when an external force in the third direction (Z) is applied to the pump (310), and may be restored to its previous state when the external force is released.

The elastic coefficient of the second spring (320) may be set to be sufficient for the volume of the pump (310) to return to its state before the external force was applied after the external force applied to the pump (310) is released. Therefore, the elastic coefficient of the second spring (320) may be set according to the size of the pump (310). In addition, the diameter, length, and thickness of the second spring (320) may be determined according to the elastic coefficient of the second spring (320) and design details of other components (e.g., the width of the pump (310) in the third direction (Z), etc.).

The second support member (340) is disposed inside the pump (310), and may fix the other end of the second spring (320) in the third direction (Z). For example, the second support member (340) is fixedly disposed on the inside of the other end of the pump (310), protrudes in the third direction (Z) from the inside of the other end of the pump (310), and may have an O-shape for the second spring (320) to be disposed inside. The inner diameter of the second support member (340) may be set so that the second spring (320) is disposed inside. However, the second support member (340) may have various structures and sizes for fixedly coupling the other end of the second spring (320) to the inside of the other end of the pump (310), as understood by those skilled in the art according to the teachings of the present invention.

The second spring (320) is a component for increasing the efficiency of pumping of the pump (310), and the second support member (340) is a component for preventing damage to the pump (310) due to the second spring (320) and preventing the second spring from bending during pumping. Accordingly, the air pressure generating unit (300) according to some embodiments may be designed with the second spring (320) and the second support member (340) omitted.

The air pressure generating unit (300) according to one embodiment generates air pressure as the volume of the space inside the pump (310) decreases when an external force is applied to the pump (310), and the air filled in the air reservoir (111) and the internal space of the pump (310) by the generated air pressure can be injected into the mixing chamber (112). The magnitude of the air pressure generated by the air pressure generating unit (300) may be determined according to the volume of the internal space of the pump (310) and the elastic coefficient of the second spring (320).

Meanwhile, the air pressure generating unit (300) according to one embodiment may be implemented to be detachable from the first main body (110). In other words, the pump (310) and the first support member (330) may have a structure for easily coupling and uncoupling from the other end of the first main body (110).

Accordingly, the powder applicator (10) according to one embodiment can be used by coupling a pump (310) and a second spring (320) designed to be suitable for the required powder spray amount. In this case, without having to manufacture the first body portion (100) and the second body portion (200) again according to the desired powder spray amount, by applying a suitable air pressure generating unit (300) among a plurality of air pressure generating units (300) designed to provide various sizes of air pressure, it is possible to provide a powder applicator (10) improved in usefulness and cost.

The filter (400) may be disposed inside the air reservoir (111). For example, the filter (400) may be disposed in contact with the inside of one end of the air reservoir (111) so as to cover the air supply passage (113). The filter (400) may have a shape substantially the same as the cross-sectional shape of the internal space of the air reservoir (111) on the X-Y plane. However, the present invention is not limited thereto, and the filter (400) may be implemented with a planar area sufficient to completely cover the air supply passage (113) on the X-Y plane and may be fixedly disposed on one end surface of the air reservoir (111).

When air pressure is generated by the air pressure generating unit (300), the filter (400) allows the air filled in the air reservoir (111) to be supplied to the mixing chamber (112) through the air supply passage (113), but can block the powder contained in the mixing chamber (112) or the powder reservoir (211) from flowing into the air reservoir (111). As a non-limiting example, the filter (400) may be implemented as a porous filter.

The discharge port (500) may comprise a powder discharge passage for discharging the powder contained in the mixing chamber (112) by the compressed air supplied by the air pressure generating unit (300). The discharge port (500) may be disposed between the spray member (600) or barrier member (700) and the mixing chamber (112).

The discharge port (500) may have one end connected to the spray member (600) or the barrier member (700) in the third direction (Z), and the other end connected to the first body portion (100). Specifically, the other end of the discharge port (500) may be fixedly coupled to the coupling portion (120). With respect to the structure of the discharge port, any known structure may be employed, and for example, reference may be made to the powder delivery device disclosed in Figs. 12 to 14 of U.S. Patent No. 10507293, but is not limited thereto.

The discharge port (500) may comprise an internal structure and an external structure. The internal structure may have a cylindrical structure in which an outer surface is fitted to an inner surface of the second sub-coupling portion (122) and a powder discharge passage is formed inside. The external structure may be formed on the outside of the internal structure and may comprise an inner surface for coupling with the first sub-coupling portion (121), and an outer surface for coupling with the spray member (600) or the barrier member (700). The internal structure and the external structure of the discharge port (500) may be changed according to the structure of the coupling portion (120).

The discharge port (500) may optionally comprise a powder trap (PT). The powder trap (PT) may be formed at the other end of the internal structure in the third direction (Z). As illustrated in Fig. 4, a separate powder trap member (PTM) is coupled to the other end of the internal structure to form the powder trap (PT), but the present invention is not limited thereto. For example, through a technology such as 3D printing, the powder trap (PT) may be formed integrally with the discharge port (500).

When the powder discharge passage of the discharge port (500) is directly connected to the internal space of the first main body (110) of the first body portion (100), the powder contained in the mixing chamber (112) can be discharged as one end of the discharge port (500) faces the direction of gravity. Accordingly, the powder trap (PT) may have a structure for preventing discharge of powder other than powder contained in the mixing chamber (112) being intentionally discharged by the air pressure generated by the air pressure generating unit (300).

For example, the powder trap (PT) may comprise a passage having an arrow-shaped facing in a direction opposite to the direction (the third direction (Z)) in which the powder is discharged, as illustrated in Figs. 5 to 7, wherein a tip portion of the arrow-shaped cross-section may have a blunt shape. That is, the passage of the powder trap (PT) allows the mixing chamber (112) and the discharge port (500) to communicate with each other, wherein a portion of the passage adjacent to the mixing chamber (112) may have a path oriented in a direction opposite to a direction in which the powder is discharged by the discharge port (500) (i.e., a direction opposite to the third direction (Z)).

Accordingly, even if the discharge port (500) is arranged along the direction in which gravity acts, the powder contained in the powder reservoir (211) cannot flow into the passage of the powder trap (PT) facing in the opposite direction of gravity, and thus, in addition to the powder being intentionally discharged when air pressure is generated by the air pressure generating unit (300), the powder can be prevented from being unintentionally discharged.

In the present specification, it is exemplified that there are two passages interconnecting with the powder trap (PT) and the mixing chamber (112), but this is not limited thereto. In another example, there may be three or more passages interconnecting with the powder trap (PT) and the mixing chamber (112), or there may be only one.

A first sealing member (PM1) may be disposed on at least a portion of the outer surface of the internal structure. The first sealing member (PM1) may provide airtightness between the internal structure of the discharge port (500) and the second sub-coupling portion (122).

The spray member (600) is configured to be coupled to the discharge port (500) to inject powder into the affected area, and may comprise a needle (610) and a needle tube (620). The needle (610) is coupled to the discharge port (500), and the needle tube (620) may be connected to the powder discharge passage of the discharge port (500) through the needle (610).

The shape of the spray member (600) may be applied to various types of spray members that are understood by those skilled in the art. For example, the spray member (600) may have various structures depending on the type and size of the affected area where the powder is sprayed, such as having a structure suitable for insertion into the patient's body through an endoscope during surgery.

The barrier member (700) may be combined with the discharge port (500) instead of the spray member (600) before the powder applicator (10) is used. The barrier member (700) can prevent the powder contained in the mixing chamber (112) from being exposed to the outside air or foreign substances such as dust from flowing into the mixing chamber (112) through the discharge port (500).

The barrier member (700) may comprise a cylindrical structure for blocking the powder discharge passage of the discharge port (500). In addition, the barrier member (700) may comprise a structure that connects to the cylindrical structure and is advantageous for the barrier member (700) to be separated from the discharge port (500). For example, the barrier member (700) may have a structure in which a cylindrical structure and a ring-shaped structure are connected in the third direction (Z), but is not limited thereto. As another example, the barrier member (700) may have various structures, such as a cylindrical structure and a hook-shaped structure connected in the third direction (Z) or a cylindrical structure and a 'T'-shaped structure connected.

A second sealing member (PM2) in the shape of an O-ring may be disposed between the spray member (600) or barrier member (700) and the discharge port (500) to provide airtightness between the two components.

Figs. 5 to 7 are cross-sectional views of a powder applicator according to one embodiment of the present invention, wherein Fig. 5 is a view showing a state in which a barrier member is coupled to a discharge port before a powder applicator according to one embodiment of the present invention is used, Fig. 6 is a view showing a state in which an external force is applied to a powder applicator according to one embodiment of the present invention, and Fig. 7 is a view showing a state after all powder contained in a powder applicator according to one embodiment of the present invention has been discharged.

Hereinafter, a method of operating a powder applicator (10) according to one embodiment will be described with reference to Figs. 5 to 7. In Figs. 5 to 7, the powder contained in the powder reservoir (211), the powder supply chamber (212), and the mixing chamber (112) is omitted for convenience of explanation, but those components may be in a state filled with powder.

First, referring to Fig. 5, a powder applicator (10) according to one embodiment may have a barrier member (700) coupled to a discharge port (500) to ensure the airtightness of the powder disposed inside the first body portion (100) and the second body portion (200) before being used by the user. The user can separate the barrier member (700) from the discharge port (500) for use and couple an spray member (600) suitable for the affected area where the powder is to be applied to the discharge port (500).

In this case, even if one end of the discharge port (500) is opened to the outside as the spray member (600) is coupled to the discharge port (500), the powder applicator (10) according to one embodiment can prevent powder from being unintentionally discharged from the powder applicator (10) in a situation where the discharge port (500) faces the direction in which gravity acts or an external action such as vibration is applied to the powder applicator (10), since the discharge port (500) includes a powder trap (PT).

Meanwhile, the powder contained in the powder reservoir (211) of the second body portion (200) is pressurized in the second direction (Y) by the first spring (230) and is supplied to the mixing chamber (112) through the powder supply chamber (212), so the mixing chamber (112) may be in a state filled with the supplied powder.

In addition, when the second body portion (200) is manufactured separately from the first body portion (100) and implemented to be combinable for use according to some embodiments, the user may couple the second body portion (200) to the first body portion (100) to use the powder applicator (10), and the mixing chamber (112) may receive powder from the powder reservoir (211) after the second body portion (200) is coupled to the first body portion (100).

Next, the user can use the handle portion (130) to support the powder applicator (10) from moving in the third direction (Z) and apply an external force to the other end of the air pressure generating unit (300) to spray the powder contained in the mixing chamber (112).

For example, when an external force acts on the other end of the pump (310), the volumes of the air reservoir (111) and the interior of the pump (310) decrease, and accordingly, air pressure is generated and air can be supplied to the mixing chamber (112). The air supplied to the mixing chamber (112) creates a flow inside the mixing chamber (112), and the powder contained in the mixing chamber (112) is transported by the airflow and passes through the powder trap (PT) to be applied to the patient's affected area through the discharge port (500) and the spray member (600).

In this case, the powder applicator (10) according to one embodiment comprises the first spring (230) that applies pressure to the powder contained inside the second body portion (200) in the second direction (Y), and is implemented such that the part filled with powder and the part supplied with air are separated from each other, so that the resistance can be reduced compared to a case where air must pass through all the powder corresponding to the total discharge amount of the powder applicator (10), which can be advantageous in improving the powder spray performance.

When the external force applied to the other end of the air pressure generating unit (300) is released, the pump (310) can be restored to its original shape before the external force was applied by the second spring (320). In this case, the airflow inside the powder applicator (10) may be generated in the direction from the mixing chamber (112) to the air reservoir (111), but since the filter (400) is disposed between the mixing chamber (112) and the air reservoir (111), it is possible to prevent the powder contained in the mixing chamber (112) from flowing into the air reservoir (111).

In addition, after at least a portion of the powder contained in the mixing chamber (112) has been sprayed by the air pressure, as illustrated in Figs. 6 and 7, the powder contained in the powder reservoir (211) is pressurized by the plunger (220) and the first spring (230), and the mixing chamber (112) can receive a supply of powder corresponding to the amount of the sprayed powder.

That is, when the powder applicator (10) is designed to spray a plurality of times, the mixing chamber (112) can be maintained filled with powder by the plunger (220) and the first spring (230) before all the powder contained in the powder reservoir (211) is discharged. Therefore, in the case of the powder applicator (10) according to one embodiment, it is advantageous to maintain the amount of powder discharged according to the plurality of times uniformly and to improve the accuracy of the timing at which the powder is sprayed.

Thereafter, the powder applicator (10) can repeat the above-described powder discharge process a preset number of times by repeatedly applying and releasing external force to the other end of the air pressure generating unit (300) a preset number of times.

Although the embodiments of the present invention have been described above with reference to the accompanying drawings, those skilled in the art will understand that the present invention may be implemented in other specific forms without changing the technical spirit or essential features of the present invention. These embodiments are not intended to limit the invention but are merely exemplary, and should be considered from an illustrative rather than a restrictive perspective. Although specific terms are used in the present specification, they are used only for the purpose of explaining the concept of the present invention and are not used to limit the meaning or the scope of the present invention as set forth in the claims. Therefore, the embodiments described above should be understood as exemplary in all respects and not restrictive.

## Claims

1. A powder applicator comprising:
a first body portion comprising an interconnected mixing chamber, an air reservoir configured to contain air, and an air supply passage disposed between the mixing chamber and the air reservoir;
a second body portion disposed on a side surface of the first body portion and supplying powder to the mixing chamber;
an air pressure generating unit disposed at the other end of the first body portion, generating air pressure in the air filled in the air reservoir and supplying air to the mixing chamber;
a filter disposed inside the air reservoir, completely covering the air supply passage, and configured to preventthe movement of powder between the mixing chamber and the air reservoir and to allow movement of air; and
a discharge port disposed at one end of the mixing chamber through which the powder is discharged,
wherein the powder contained in the mixing chamber is transported by an airflow generated by the air pressure of the air pressure generating unit and discharged through the discharge port.

2. The powder applicator according to claim 1,
wherein the first body portion comprises:
a first main body comprising the mixing chamber, the air reservoir, and the air supply passage;
a coupling portion disposed at one end of the first main body and having a structure for coupling with the discharge port; and
a handle portion disposed adjacent to the coupling portion and having a shape protruding from a side surface of the first main body.

3. The powder applicator according to claim 1,
wherein the second body portion comprises:
a second main body comprising a powder reservoir;
a closing member closing the other end of the second main body;
a plunger disposed inside the second main body and configured to sealbetween the powder reservoir and a space adjacent to the other end of the second main body; and
a first spring having one end coupled to the plunger and the other end coupled to the closing member.

4. The powder applicator according to claim 3,
wherein the second main body further comprises a powder supply chamber in communication with the powder reservoir,
the powder supply chamber is disposed inside the mixing chamber,
the powder contained in the powder reservoir is supplied to the mixing chamber through the powder supply chamber, and
the planar area of the opening through which the powder is supplied from the powder supply chamber to the mixing chamber is smaller than the planar area of the powder reservoir.

5. The powder applicator according to claim 3,
wherein the plunger is biased by the first spring to apply pressure to the powder contained in the powder reservoir.

6. The powder applicator according to claim 1,
wherein the air pressure generating unit comprises:
a pump having a structure that folds in a corrugated manner when an external force is applied; and
a first support member fixedly coupled together with the pump to the other end of the first body portion.

7. The powder applicator according to claim 6,
wherein the air pressure generating unit further comprises:
a second spring disposed between the first support member and the other end of the pump; and
a second support member disposed between the second spring and the pump to prevent the second spring from bending when an external force is applied to the pump.

8. The powder applicator according to claim 6,
wherein the air pressure generating unit is detachable from the first body portion.

9. The powder applicator according to claim 1,
further comprising a barrier member coupled to the discharge port to block contact between the powder and the external environment.

10. The powder applicator according to claim 1,
wherein the first body portion and the second body portion are manufactured separately and are combinable.
